(19) Europäisches Patentamt
European Patent Office
Office européen des brevets

(11) EP 1 198 219 B1

(12) **EUROPÄISCHE PATENTSCHRIFT**

(45) Veröffentlichungstag und Bekanntmachung des Hinweises auf die Patenterteilung:
**18.05.2005 Patentblatt 2005/20**

(21) Anmeldenummer: **01956204.0**

(22) Anmeldetag: **24.03.2001**

(51) Int Cl.[7]: **A61K 7/09**

(86) Internationale Anmeldenummer:
**PCT/EP2001/003388**

(87) Internationale Veröffentlichungsnummer:
**WO 2001/074318 (11.10.2001 Gazette 2001/41)**

(54) **MITTEL UND VERFAHREN ZU DAUERHAFTEN HAARVERFORMUNG AUF BASIS VON 2-MERCAPTOPROPIONSÄUREAMIDEN SOWIE VERFAHREN ZU DEREN HERSTELLUNG**

METHOD AND AGENTS ON THE BASIS OF 2-MERCAPTOPROPIONIC ACID AMIDES FOR DURABLE HAIR FORMING AND A METHOD FOR PRODUCING SAME

AGENT ET PROCEDE POUR LE PERMANENTAGE DURABLE DES CHEVEUX A BASE D'AMIDES DE L'ACIDE 2-MERCAPTOPROPIONIQUE ET PROCEDE POUR LEUR PRODUCTION

(84) Benannte Vertragsstaaten:
**AT BE CH CY DE DK ES FI FR GB GR IE IT LI LU MC NL PT SE TR**

(30) Priorität: **01.04.2000 DE 10016406**

(43) Veröffentlichungstag der Anmeldung:
**24.04.2002 Patentblatt 2002/17**

(73) Patentinhaber: **Wella Aktiengesellschaft**
**64274 Darmstadt (DE)**

(72) Erfinder:
- **DANNECKER, Beate**
  **71034 Böblingen (DE)**
- **SCHWEITZER, Ursus**
  **64743 Beerfelden (DE)**
- **LANG, Günther**
  **64354 Reinheim (DE)**
- **ORTMANN, Regina**
  **35043 Marburg (DE)**
- **HANEFELD, Wolfgang**
  **35037 Marburg/Lahn (DE)**

(56) Entgegenhaltungen:
**WO-A-91/10421**
**CH-A- 519 620**
**FR-A- 2 465 478**
**US-A- 5 068 102**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 1 198 219 B1

## Beschreibung

Die vorliegende Erfindung betrifft ein Mittel zur dauerhaften Haarverformung, welches als keratinreduzierenden Wirkstoff bestimmte neue 2-Mercaptopropionsäureamide enthält sowie ein Verfahren zur dauerhaften Haarverformung unter Verwendung derartiger Mittel.

Bekanntlich beruht die klassische Technik zur Durchführung einer dauerhaften Haarverformung auf zwei Behandlungsschritten: Im ersten Schritt werden die Cystin-Disulfid-Brücken des Haarkeratins durch Einwirken eines Mittels, welches einen reduzierenden Wirkstoff enthält (Verformungsmittel), geöffnet. Sodann wird das Haar in die gewünschte Form gebracht. In einem zweiten Schritt werden Cystin-Disulfid-Bindungen unter Verwendung eines Fixiermittels, d.h. eines einen oxidierenden Wirkstoff enthaltenden Mittels, wieder geschlossen.

Als klassisches Dauerwellreduktionsmittel wird, wie die Pionierarbeiten in den deutschen Patentschriften 948 186 und 972 424 zeigen, die Thioglykolsäure, z.B. als Ammonium- oder Monoethanolaminsalz, verwendet. Weitere übliche reduzierende Wirkstoffe sind anorganische Sulfite, 2-Mercapto-propionsäure (Thiomilchsäure), 3-Mercapto-propionsäure, bestimmte Mercaptocarbonsäureester, Cystein und Derivate dieser Verbindungen.

Alle diese Mittel weisen jedoch eine Reihe von Nachteilen auf. Alkalisch eingestellte Zubereitungen auf Basis der Mercaptocarbonsäuren zeigen trotz ausreichender Wirkung eine Haarschädigung, die sich beispielsweise in vermehrt auftretendem Haarbruch äußert. Vielfach belasten diese Mittel auch in unerwünschter Weise die Kopfhaut.

Schließlich erfordert der unangenehme Geruch der verwendeten Reduktionsmittel eine intensive Parfümierung der Produkte. Durch Verwendung von 2-Mercapto-propionsäure (Thiomilchsäure) ist man in der Lage, einige der erwähnten Probleme zu lösen. Allerdings zeichnet sich die Thiomilchsäure im Vergleich zur allgemein verwendeten Thioglykolsäure durch eine schwächere Umformung aus.

Die Mercaptocarbonsäureester, welche eine Haarverformung auch bei niedrigeren pH-Werten ermöglichen, sind bezüglich ihrer Hautverträglichkeit sowie ihres Sensibilisierungsrisikos nicht zufriedenstellend. Anstelle der Mercaptocarbonsäureester wurden auch Mercaptocarbonsäureamide wie Thioglykolsäureamid oder alkyl- bzw. hydroxyalkylsubstituierte Amide verwendet. Derartige Verbindungen sind aus der Patentliteratur WO-A-91/10421 und EP-A-0 455 457 bekannt. Diese Stoffe haben, wie die Carbonsäureester, ein hohes Umformungspotential auch bei niedrigen pH-Werten, sind jedoch in Bezug auf die Sensibilisierung noch kritischer als die Ester.

Es wurde nun überraschend gefunden, daß sich die vorstehenden Nachteile durch die Verwendung bestimmter 2-Mercaptopropionsäureamide vermeiden lassen und daß diese über ein stärkeres Umformungspotential als Thiomilchsäure verfügen.

Gegenstand der vorliegenden Erfindung ist daher ein Mittel zur dauerhaften Verformung der Haare, welches dadurch gekennzeichnet ist, daß es als keratinreduzierenden Wirkstoff ein 2-Mercaptopropionsäureamid der allgemeinen Formel

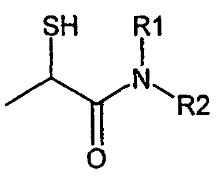

(I)

enthält, in der $R_1$ und $R_2$ unabhängig von einander jeweils die Bedeutung hat H, geradkettiger oder verzweigter Alkylrest mit 1 - 4 C-Atomen, geradkettiger oder verzweigter Mono- oder Dihydroxyalkylrest mit 2 - 4 C-Atomen, geradkettiger oder verzweigter Alkoxyalkylrest oder Alkylaminoalkylrest mit 2 bis 8 C-Atomen, Morpholinoalkyl mit 2 - 3 C-Atomen im Alkylrest, unter der Voraussetzung, daß $R_1$ und $R_2$ nicht gleichzeitig H bedeuten, oder $R_1$ und $R_2$ gemeinsam mit dem Stickstoffatom einen fünf- oder sechsgliedrigen heterocyclischen Ring bilden, der noch ein weiteres Heteroatom, insbesondere N oder O, enthalten kann und mit einer oder zwei OHund/oder Methyl-Gruppen substituiert sein kann.

Bevorzugte Verbindungen der Formel (I) sind solche, in denen $R_1$ und $R_2$ unabhängig voneinander die Bedeutung H, $-CH_3$, $-CH_2CH_2CH_3$, $-CH(CH_3)_2$, $-CH_2CH_2OH$, $-CH_2CH(OH)CH_3$, $-CH(CH_2CH_3)(CH_2OH)$, $-CH_2CH_2CH_2N(CH_3)_2$, $-CH_2CH_2OCH_3$, $-CH_2CH_2CH_2OCH_3$, $-CH_2CH_2N(CH_2CH_3)_2$, $-CH_2CH_2N(CH_3)_2$ oder Morpholinoalkyl. mit 2 - 3 C-Atomen im Alkylrest, haben, unter der Voraussetzung, daß $R_1$ und $R_2$ nicht gleichzeitig H bedeuten.

Besonders bevorzugte Verbindungen sind solche der Formeln

(II) 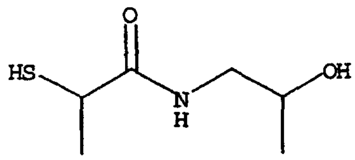 oder (III) 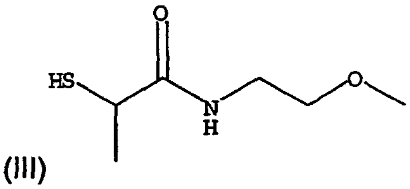

Die Herstellung der erfindungsgemäßen 2-Mercaptopropionsäureamide erfolgt durch Umsetzung der entsprechenden Amine mit 2-Mercaptopropionsäure-methylester unter Schutzgasatmosphäre, Extraktion in geeignetem Lösungsmittel und anschließende Kurzwegdestillation.

Die 2-Mercaptopropionsäureamide der Formel (I) werden in dem gebrauchsfertigen Mittel zur dauerhaften Haarverformung in einer Menge von 3 bis 28 Gewichtsprozent, vorzugsweise 5 bis 21 Gewichtsprozent, eingesetzt.

Die erfindungsgemäßen 2-Mercaptopropionsäureamide können in einer weiteren Ausführungsform der Erfindung auch im Gemisch mit anderen, bekannten Thiolen wie Thioglykolsäure, Thiomilchsäure, Cystein, Cysteamin und Alkyl- oder Acylcysteaminen oder Sulfiten eingesetzt werden.

Die gebrauchsfertigen Haarverformungsmittel besitzen bevorzugt einen pH-Wert von 6,5 bis 9,5 besonders bevorzugt von 6,5 bis 8,5. Als Alkalisierungsmittel bzw. als Mittel zur Einstellung des pH-Wertes kommen insbesondere Ammoniak oder Natronlauge, aber auch alle anderen wasserlöslichen, physiologisch verträglichen Salze von organischen und anorganischen Basen, wie z.B. Ammoniumhydrogencarbonat, in Betracht.

Das Verformungsmittel kann sowohl ein- als auch zweikomponentig verpackt angeboten werden. Liegt es zweikomponentig vor, dann werden die beiden Komponenten unmittelbar vor dem Gebrauch miteinander vermischt. Das Mittel kann sowohl in Form einer wäßrigen Lösung oder einer Emulsion als auch in verdickter Form auf wäßriger Basis, insbesondere als Creme, Gel, Schaum oder Paste vorliegen.

Selbstverständlich kann das Verformungsmittel alle für derartige Mittel üblichen und bekannten Zusatzstoffe, zum Beispiel Verdickungsmittel, wie beispielsweise Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester, ferner Trübungsmittel, wie zum Beispiel Polyethylenglykolester; Alkohole, wie zum Beispiel Ethanol, Propanol, Isopropanol und Glycerin; Zucker wie z. B. D-Glucose; Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 1 bis 30 Gewichtsprozent sowie zur Vermeidung einer Überkrausung der Haare Dithioverbindungen, beispielsweise Dithiodiglykolsäure, Dithiomilchsäure, die Dithiole der Verbindungen nach Formel (I) oder die jeweiligen Salze der Dithiole, zugesetzt werden.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte und gleichmäßige Umformung vom Haaransatz bis zur Haarspitze, ohne allergische oder sensibilisierende Reaktionen hervorzurufen.

Die vorliegende Erfindung betrifft weiterhin ein Verfahren zur dauerhaften Haarverformung, bei dem man die Haare bevor und/oder nachdem man sie in die gewünschte Form bringt, mit einem Verformungsmittel behandelt, mit Wasser spült, sodann oxidativ behandelt, mit Wasser spült, gegebenenfalls zur Wasserwelle legt und sodann trocknet, welches dadurch gekennzeichnet ist, daß als Verformungsmittel die vorstehend beschriebenen erfindungsgemäßen Mittel verwendet werden.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne

Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung im aufgewickelten oder abgewickelten Zustand kann jedes beliebige, für eine derartige Behandlung geeignetes Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.% in dem Nachbehandlungsmittel enthalten sein.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung näher erläutern.

## SYNTHESEMETHODEN:

### Herstellung der 2-Mercaptopropionsäureamide nach Methode A

In einem 500 ml-Dreihalskolben werden 2 bis 3 mol des jeweiligen Amins vorgelegt. Unter Kühlung mit einem Wasserbad wird langsam 1 mol 2-Mercaptopropionsäure-methylester so zugetropft, daß die Temperatur 30°C nicht übersteigt. Der Ansatz wird mit Argon durchspült und solange gerührt (ggf. unter leichtem Erwärmen), bis der Ester quantitativ umgesetzt ist (Kontrolle durch IR-Spektroskopie). Das Gemisch wird unter Eiskühlung mit 32 %iger Salzsäure angesäuert (pH 2 - 4) und mit Ethylacetat oder t-Butylmethylether erschöpfend extrahiert. Das Lösungsmittel wird am Umlaufverdampfer im Vakuum abdestilliert, der Rückstand durch Zugabe von Natronlauge auf pH 7,0 eingestellt und nochmals mit Ethylacetat oder t-Butylmethylether erschöpfend extrahiert. Die vereinigten Fraktionen werden über Natriumsulfat getrocknet und eingeengt. Der vorliegende Rückstand wird mittels einer Kurzwegdestillationsapparatur (Gleichstromdestillation) bei ca. 0,02 mbar destilliert oder in einem geeigneten Lösungsmittel umkristallisiert zum weitestgehend reinen Produkt oder durch Säulenchromatographie gereinigt

### Herstellung der 2-Mercaptopropionsäureamide nach Methode B

In einem 500 ml-Dreihalskolben werden 2 mol des jeweiligen Amins vorglegt. Unter Kühlung mit einem Wasserbad wird langsam 1 mol 2 Mercaptopropionsäuremethylester derart zugetropft, daß die Temperatur 30°C nicht übersteigt. Der Ansatz wird mit Argon durchspült und solange gerührt (ggf. unter leichtem Erwärmen), bis der Ester quantitativ umgesetzt ist (Kontrolle durch IR-Spektroskopie).
Anschließend wird das überschüssige Amin bei ca. 1 mbar abdestilliert. Der vorliegende Rückstand wird bei ca. 0,08 mbar über eine Vigreuxkolonne destilliert oder mittels einer Kurzwegdestillationsapparatur (Gleichstromdestillation) bei ca. 0,02 mbar destilliert oder in einem geeigneten Lösungsmittel umkristallisiert zum weitestgehend reinen Produkt.

### Herstellung der 2-Mercaptopropionsäureamide nach Methode C

In einem 1 l-Dreihalskolben werden 2 mol des jeweiligen Amins vorgelegt. Unter Kühlung mit einem Wasserbad werden langsam 1 mol 2-Chlorpropionsäuremethylester derart zugetropft, daß die Temperatur 30°C nicht übersteigt. Der Ansatz wird solange gerührt, bis der Ester quantitativ umgesetzt ist (Kontrolle durch IR-Spektroskopie). Anschließend wird unter Kühlung mit einem Wasserbad 1 mol Kalium-Ethylxanthogenat löffelweise zugegeben. Der Ansatz wird noch zwei Stunden lang unter Kühlung mit einem Wasserbad und weitere zwölf Stunden lang bei Raumtemperatur gerührt. Dann werden 500 ml 25 %ige Ammoniaklösung zugegeben und weitere 24 Stunden lang gerührt.

Die ammoniakalische Lösung wird mit Ethylacetat ausgeschüttelt. Die wässerige Phase wird unter Eiskühlung

mit 32 %iger Salzsäure angesäuert (pH 2 - 4) und mit Ethylacetat erschöpfend extrahiert. Das Lösungsmittel wird am Umlaufverdampfer im Vakuum abdestilliert, der Rückstand durch Zugabe von Natronlauge auf pH 7,0 eingestellt und nochmals mit Ethylacetat erschöpfend extrahiert. Die vereinigten Fraktionen werden über Natriumsulfat getrocknet und eingeengt. Der vorliegende Rückstand wird mittels einer Kurzwegdestillationsapparatur (Gleichstromdestillation) bei ca. 0,02 mbar destilliert oder in einem geeigneten Lösungsmittel umkristallisiert zum weitestgehend reinen Produkt.

**Herstellung der 2-Mercaptopropionsäureamide nach Methode D**

In einem 1 l-Dreihalskolben wird 1 mol des jeweiligen Amins mit 500 ml 2N-Natronlauge versetzt und in einem Eis-Kochsalzbad auf 0°C gekühlt. 1 Mol 2-Chlorpropionsäurechlorid wird so zugetropft, daß die Temperatur 5°C nicht übersteigt. Der Ansatz wird drei Stunden lang bei Raumtemperatur kräftig gerührt. Anschließend wird unter Kühlung mit einem Wasserbad 1 mol Kalium-Ethylxanthogenat löffelweise zugegeben. Der Ansatz wird noch zwei Stunden lang unter Kühlung mit einem Wasserbad und weitere zwölf Stunden lang bei Raumtemperatur gerührt. Das Gemisch wird mit 32 %iger Salzsäure solange angesäuert, bis sich ein gelbes Öl abscheidet. Dieses Öl wird abgetrennt, mit 500 ml 25 %iger Ammoniaklösung versetzt und weitere 24 Stunden lang gerührt.

Die ammoniakalische Lösung wird mit Ethylacetat ausgeschüttelt. Die wässerige Phase wird unter Eiskühlung mit 32 %iger Salzsäure angesäuert (pH 2 - 4) und mit Ethylacetat erschöpfend extrahiert. Das Lösungsmittel wird am Umlaufverdampfer im Vakuum abdestilliert, der Rückstand durch Zugabe von Natronlauge auf pH 7,0 eingestellt und nochmals mit Ethylacetat erschöpfend extrahiert. Die vereinigten Fraktionen werden über Natriumsulfat getrocknet und eingeengt. Der vorliegende Rückstand wird mittels einer Kurzwegdestillationsapparatur (Gleichstromdestillation) bei ca. 0,02 mbar destilliert oder in einem geeigneten Lösungsmittel umkristallisiert zum weitestgehend reinen Produkt.

**Vergleich der Wellstabilitäten**

Die Wellwirksamkeit der 1-Mercaptopropionsäureamide wurde unter Verwendung von Glycerinmonothioglykolat als Vergleichssubstanz mit Hilfe von Wellösungen bei pH = 7, 8 und 9 bestimmt. Hierzu wurden 16,5 Zentimeter lange, vorgebleichte und damit geschädigte Zählhaarsträhnen (bestehend aus ca. 100 Haaren) aus mitteleuropäischem Haar naß auf genormte Spiralwickler (Innendurchmesser. 3 Millimeter) aufgewickelt und nach dem Konditionieren im Klimaraum (Temperatur: 20°C; Luftfeuchte: 65%) mit einer 87 mmol/100g enthaltenden, auf den jeweiligen pH-Wert eingestellten Lösung der Reduktionsmittel behandelt. Die Auftragemenge an Wellflüssigkeit wurde über das Verhältnis 1 : 1,2 errechnet (1g Haar: 1,2 ml Wellflüssigkeit). Als Einwirkzeit wurden 20 Minuten gewählt; die Einwirktemperatur betrug 50 Grad Celsius. Anschließend wurden die Haare mit einer peroxidhaltigen Fixierung fixiert, getrocknet und nach dem Abwickeln vier Stunden lang in Wasser (Wasserbadtemperatur: 40°C) ausgehängt.

Die Wellstabilität (WSN) errechnet sich gemäß folgender Formel:

$$\text{Wellstabilität in \%} = \frac{l_o - l_t}{l_o - l_1} \times 100$$

$l_o$ = Gesamtlänge der nicht umgeformten, gestreckten Strähne (16,5 Zentimeter)
$l_t$ = Länge der abgewickelten, ausgehängten Strähne nach 240 Minuten
$l_1$ = Länge der umgeformten, aufgewickelten Strähne
(bei einem Wickelinnendurchmesser von 3 mm beträgt $l_1$ = 35 mm)

Als Standard wurden Strähnchen mit einer entsprechend auf pH 9 eingestellten Glycerinmonothioglykolat-Lösung behandelt. Die vorstehend in Tabelle 1 angegebenen normierten Wellstabilitäten beziehen sich auf diese Standardlösung (pH = 9), deren Wellstabilität (WSN) auf 100 Prozent gesetzt wurde.

**Beispiel 1: Herstellung von N-(2-Hydroxypropyl)-2-mercaptopropionsäureamid nach Methode A**

150 g (2 mol) 1-Amino-2-propanol und 120 g (1 mol) 2-Mercaptopropionsäure-methylester werden nach Methode A umgesetzt. Das Produkt wird durch Destillation bei 110°C und 0,02 Torr gereinigt. Die Ausbeute beträgt 83,6 g (51 % der Theorie).

Analytik:

a) $^{1}$H-NMR ($CDCl_3$):

| | | |
|---|---|---|
| δ (ppm) = | 7,02 | (bs, 1 H, NH) |
| | 3,96 | (m, 1 H, CH-OH) |
| | 3,45 | (m, 2 H, CH2-NH) |
| | 3,15 | (m, 1 H, CH-SH) |
| | 3,01 | (bs, 1 H, OH) |
| | 2,12 | (bs, 1 H, SH) |
| | 1,55 | (d, 3 H, SH-CH-$CH_3$) |
| | 1,21 | (d, 3 H, OH-CH-$CH_3$) |

b) $^{13}$C-NMR ($CDCl_3$):

| | | |
|---|---|---|
| δ (ppm) = | 174,13 | (CO) |
| | 67,20 | (CH-OH) |
| | 47,25 | (NH-$CH_2$) |
| | 38,14 | (HS-CH) |
| | 22,20 | (HS-CH-$CH_3$) |
| | 20,91 | (HO-CH-$CH_3$) |

c) MS (70 eV, EI, 150 °C)

m/z (%) = (M+)= 163 (42)
119 (100), 106 (41), 102 (28), 86 (97), 85 (23), 84 (63), 61 (47), 58 (33), 56 (27)

d) Thioltitration: 99,1 %

e)

| Elementaranalyse: $C_6H_{13}NO_2S$ (MG: 163,24 g/mol) | | | | |
|---|---|---|---|---|
| Ber. | C 44,15 | H 8,03 | N 8,58 | S 19,64 |
| Gef. | C 43,44 | H 8,06 | N 8,40 | S 19,57 |

f) IR (NaCl-Gläser):

| | |
|---|---|
| 3307 $cm^{-1}$ | (OH) |
| 2976 $cm^{-1}$ + 2931 cm-1 | ($CH_2$) |
| 2543 $cm^{-1}$ | (SH) |
| 1653 $cm^{-1}$ | (N-monosubstituiertes Amid) |
| 1556 $cm^{-1}$ | (N-monosubstituiertes Amid) |

| | | |
|---|---|---|
| HPLC | relative Reinheit | 97,8 Flächenprozent |
| | Retentionszeit | 3,0 min |
| | Säule | Adsorbosphere C18 5U, 250 mm x 4,6 mm |
| | Fließmittel | Acetonitril : Puffer = 25 : 75 (Puffer: 4 g Kaliumdihydrogenphosphat + 0,8 g Octansulfonsäure-Natriumsalz + 2 ml Phophorsäure pro 1 l Wasser für HPLC) |
| | Flußrate | 1 ml/min |
| | Wellenlänge | 202 nm |

g) UV-max: 197 nm (Acetonitril : Puffer = 25 : 75)

h) pKs-Wert: 8,37

i) Schmelzpunkt: 40°C

Die nachfolgende Tabelle zeigt, daß die Wellwirksamkeiten der erfindungsgemäßen 2-Mercaptopropionsäureamide bei pH-Werten von 7, 8 und 9 höher sind als bei Thiomilchsäure.

| Aminkomponente Propionsäureamid | Elementaranalyse ber./gef: | HPLC (Flächenprozent) | Siedepunkt | WSN pH = 7 | WSN pH = 8 | WSN pH = 9 | Synthese-methode | Ausbeute |
|---|---|---|---|---|---|---|---|---|
| 1-Amino-2-propanol | C: 44,15 %, H: 8,03 %<br>N: 8,58 %, S 19,64 %<br>C: 43,44 %, H: 8,06 %<br>N: 8,40 %, S: 19,57 % | 97,8 % | (Schmelzpunkt: 40 °C) | 78 % | 94 % | 94 % | A | 51 % |
| N-(2-Hydroxypropyl)-2-mercapto-propionsäureamid | C: 43,42 %, H: 7,71 %<br>N: 8,44 %, S: 19,65 % | 93,0 % | 110°C (0,02 Torr) | | | | C | 34 % |
| 2-Amino-2-methyl-1-propanol | C: 47,43 %, H: 8,53 %<br>N: 7,90 %, S 18,09 %<br>C: 47,70 %, H: 8,40 %<br>N: 8,01 %, S: 17,92 % | 97,1 % | (Schmelzpunkt: 70°C) | 79 % | 91 % | 94 % | A | 28 % |
| N-(2-Hydroxy-t-butyl)-2-mercapto-propionsäureamid | C: 47,49 %, H: 8,36 %<br>N: 8,05 %, S: 17,85 % | 99,4 % | (Schmelzpunkt: 63°C) | | | | C | 26 % |
| 2-Amino-1-butanol<br>N-(1-Hydroxymethylpropyl)-2-mercaptopropionsäureamid | C: 47,43 %, H: 8,53 %<br>N: 7,90 %, S 18,09 %<br>C: 47,51 %, H: 7,89 %<br>N: 7,75 %, S: 17,94 % | 96,9 % | (Schmelzpunkt: 56°C) | 73 % | 88 % | 85 % | A | 20 % |
| 2-Amino-2-methyl-1,3-propandiol<br>[1,1-Bis(hydroxymethyl)-ethyl]-2-mercapopropionsäureamid | C: 43,50 %, H: 7,82 %<br>N: 7,25 %, S 16,59 %<br>C: 43,47 %, H: 7,60 %<br>N: 7,22 %, S: 16,17 % | 93,0 % | (Schmelzpunkt: 138°C) | | | | A | 20 % |
| Ethanolamin<br>N-(2-Hydroxyethyl)-2-mercapto-propionsäureamid | C: 40,25 %, H: 7,43 %<br>N: 9,39 %, S 21,49 %<br>C: 40,16 %, H: 7,30 %<br>N: 9,43 %, S: 21,06 % | 100 % | (Schmelzpunkt: 56 °C) | 84 % | 106 % | 103 % | A | 32 % |

| Aminkomponente<br>Propionsäureamid | Elementaranalyse<br>ber./gef: | HPLC<br>(Flächen-prozent) | Siedepunkt | WSN<br>pH = 7 | WSN<br>pH = 8 | WSN<br>pH = 9 | Synthese-methode | Ausbeute |
|---|---|---|---|---|---|---|---|---|
| Methylaminoethanol<br><br>N-Methyl,N-(2-hydroxyethyl)-2-mercapto-propionsäureamid | C: 44,15 %, H: 8,03 %<br>N: 8,58 %, S 19,64 %<br>C: 44,33 %, H: 8,06 %<br>N: 8,60 %, S: 19,84 % | 97,3 % | 95°C<br>(0,02 Torr) | 72 % | 91 % | 91 % | A | 38 % |
| Ethylaminoethanol | C: 47,43 %, H: 8,53 %<br>N: 7,90 %, S 18,09 %<br>C: 47,23 %, H: 8,16 %<br>N: 8,01 %, S: 17,84 % | 94,7 % | 95°C<br>(0,02 Torr) | 94 % | 83 % | 114% | A | 68 % |
| N-Ethyl,N-(2-hydroxyethyl)-2-mercapto-propionsäureamid | C: 47,36 %, H: 8,28 %<br>N: 7,88 %, S: 17,88% | 96,4 % | 95°C<br>(0,02 Torr) | | | | C | 29 % |
| Propylaminoethanol<br><br>N-Propyl,N-(2-hydroxyethyl)-2-mercapto-propionsäureamid | C: 50,23 %, H: 8,96 %<br>N: 7,32 %, S 16,76 %<br>C: 50,10 %, H: 8,70 %<br>N: 7,33 %, S: 16,81 % | 95,5 % | 80°C<br>(0,02Torr) | 77 % | 89 % | 99 % | A | 48 % |
| Isopropylaminoethanol<br><br>N-Isopropyl,N-(2-hydroxyethyl)-2-mercaptopropionsäureamid | C: 50,23 %, H: 8,96 %<br>N: 7,32 %, S 16,76 %<br>C: 49,97 %, H: 8,52 %<br>N: 7,24 %, S: 16,80 % | 98,0 % | 100°C<br>(0,02 Torr) | 65 % | 88 % | 109 % | A | 54 % |
| 3-Hydroxypiperidin<br><br>N,N-[3-Hydroxy-pentamethylen]-2-mercaptopropionsäureamid | C: 50,77 %, H: 7,99 %<br>N: 7,40 %, S 16,94 %<br>C: 49,62 %, H: 7,99 %<br>N: 7,38 %, S: 17,47 % | 97,6 %<br>(Thioltitr.) | 110°C<br>(0,02 Torr) | 79 % | 94 % | 89 % | D | 21 % |

| Aminkomponente<br>Propionsäureamid | Elementaranalyse<br>ber./gef: | HPLC<br>(Flächenprozent) | Siedepunkt | WSN<br>pH = 7 | WSN<br>pH = 8 | WSN<br>pH = 9 | Synthese-methode | Ausbeute |
|---|---|---|---|---|---|---|---|---|
| 2-Methoxyethylamin<br><br>N-(2-Methoxyethyl)-2-mercaptopropion-säureamid | C: 44,15 %, H: 8,03 %<br>N: 8,58 %, S 19,64 %<br>C: 44,05 %, H: 7,95 %<br>N: 8,47%, S: 19,52 % | 98,3 % | 85°C<br>(0,08 mbar) | 87 % | 99 % | 106 % | A | 67 % |
| 3-Methoxypropylamin<br><br>N-(3-Methoxypropyl)-2-mercaptopropion-säureamid | C: 47,43 %, H: 8,53 %<br>N: 7,90 %, S 18,09 %<br>C: 47,16 %, H: 8,24 %<br>N: 7,80 %, S: 17,94 % | 98,4 % | 97°C<br>(0,08 mbar) | 79 % | 92 % | 91 % | A | 69 % |
| Tetrahydrofurfurylamin<br><br>N-Tetrahydrofurfuryl-2-mercaptopropion-säureamid | C: 50,77 %, H: 7,99 %<br>N: 7,40 %, S 16,94 %<br>C: 50,75 %, H: 7,99 %<br>N: 7,35 %, S: 16,56 % | 98,6 %<br>(Thioltitration) | 82°C<br>(0,06 mbar) | 85 % | 88 % | 94 % | A | 75 % |
| 2-Amino-1-methoxypropan<br><br>N-(1-Methoxy-2-propyl)-2-mercaptopropion-säureamid | C: 47,43 %, H: 8,53 %<br>N: 7,90 %, S 18,09 %<br>C: 47,52 %, H: 8,36 %<br>N: 8,22 %, S: 18,26 % | 98,2 % | 60°C<br>(0,06 mbar) | 91 % | 95 % | 99 % | D | 15 % |
| Bis-(2-methoxyethyl)-amin<br><br>N,N-Bis-(2-Methoxyethyl)-2-mercapto-propionsäureamid | C: 48,84 %, H: 8,65 %<br>N: 6,33 %, S 14,49 %<br>C: 48,71 %, H: 8,64 %<br>N: 6,58 %, S: 14,65 % | 97,5 % | 85°C<br>(0,06 mbar) | 78 % | 93 % | 91 % | D | 32 % |
| Morpholin<br><br>N,N-3-Oxa-pentamethylen-2-mercapto-propionsäureamid | C: 47,98 %, H: 7,48 %<br>N: 7,99 %, S 18,30 %<br>C: 47,74 %, H: 7,37 %<br>N: 7,78 %, S: 17,98 % | 98,2 % | 80°C<br>(0,02 Torr) | 83 % | 93 % | 96 % | A | 27 % |

| Aminkomponente<br>Propionsäureamid | Elementaranalyse<br>ber./gef: | HPLC<br>(Flächen-prozent) | Siedepunkt | WSN<br>pH = 7 | WSN<br>pH = 8 | WSN<br>pH = 9 | Synthese-<br>methode | Ausbeute |
|---|---|---|---|---|---|---|---|---|
| N-Methylpiperazin<br><br>N,N-(4-Methylaza-pentamethylen)-2-mercaptopropionsäureamid | C: 51,03 %, H: 8,56 %<br>N: 14,88 %, S 17,03 %<br>C: 50,84 %, H: 8,34 %<br>N: 14,80 %, S: 16,43 % | 97,5 % | 98°C<br>(0,1 mbar) | 78 % | 86 % | 78 % | B | 15 % |
| 3-Dimethylaminopropylamin<br><br>N-(3-Dimethylaminopropyl-2-mercaptopropionsäureamid | C: 50,49 %, H: 9,53 %<br>N: 14,72 %, S 16,85 %<br>C: 50,12 %, H: 9,31 %<br>N: 14,78 %, S: 16,85 % | 98,1 % | 84°C<br>(0,05 mbar) | 72 % | 83 % | 95 % | B | 72 % |
| 3-Diethylaminopropylamin<br><br>N-(3-Diethylaminopropyl)-2-mercapto-propionsäureamid | C: 55,00 %, H:10,16 %<br>N: 12,83 %, S 14,68 %<br>C: 54,53 %, H: 9,68 %<br>N: 12,55 %, S: 14,40 % | 97,4 % | 65°C<br>(0,02 Torr) | 77 % | 73 % | 81 % | B | 81 % |
| 3-(Methylamino)-propylamin<br><br>N-(3-Methylaminopropyl)-2-mercapto-propionsäureamid | C: 47,69 %, H: 9,15 %<br>N: 15,89 %, S 18,19 %<br>C: 47,75 %, H: 9,10 %<br>N: 15,20 %, S: 18,06 % | 98,8 %<br>(Thioltitr.) | (Schmelz-punkt99°C) | 55 % | 73 % | 78 % | B | 45 % |
| 2-Dimethylaminoethylamin<br><br>N-(2-Dimethylaminoethyl)-2-mercapto-propionsäureamid | C: 47,69 %, H: 9,15 %<br>N: 15,89 %, S 18,19 %<br>C: 47,05 %, H: 9,05 %<br>N: 14,95 %, S: 17,80 % | 98,4 % | 83°C<br>(0,06 mbar) | 81 % | 87 % | 83 % | B | 79 % |

| Aminkomponente<br>Propionsäureamid | Elementaranalyse<br>ber./gef: | HPLC<br>(Flächenprozent) | Siedepunkt | WSN<br>pH = 7 | WSN<br>pH = 8 | WSN<br>pH = 9 | Synthese-<br>methode | Ausbeute |
|---|---|---|---|---|---|---|---|---|
| 2-Diethylaminoethylamin<br><br>N-(2-Diethylaminoethyl)-2-<br>mercaptopropionsäureamid | C: 52,90 %, H: 9,87 %<br>N: 13,71 %, S 15,69 %<br>C: 52,84, %, H: 9,72 %<br>N: 13,38 %, S: 15,36 % | 99,3 % | 74°C<br>(0,06 mbar) | 71 % | 80 % | 77 % | B | 81 % |
| 2-(Ethylamino)-ethylamin<br><br>N-(2-Ethylaminoethyl)-2-<br>mercaptopropionsäureamid | C: 47,69 %, H: 9,15 %<br>N: 15,89 %, S 18,19 %<br>C: 47,69 %, H: 8,86 %<br>N: 15,78 %, S: 18,19 % | 98,1 % | (Schmelzpunkt:<br>82°C) | 57 % | 82% | 60 % | B | 53 % |
| Ethylendiamin<br><br>N-(2-Aminoethyl)-2-mercapto-<br>propionsäureamid | C: 40,51 %, H: 8,16 %<br>N: 18,90 %, S 21,63 %<br>C: 40,52 %, H: 8,06 %<br>N: 17,92 %, S: 21,41 % | 96,7 %<br>(Thioltitr.) | (Schmelzpunkt:<br>121°C) | 61 % | 81 % | 87% | B | 39 % |
| 4-(2-Aminoethyl)-morpholin<br><br>N-(2-Morpholinoethyl)-2-<br>mercaptopropionsäureamid | C: 49,51 %, H: 8,31 %<br>N: 12,83 %, S 14,68 %<br>C: 49,41 %, H: 7,94 %<br>N: 12,65 %, S: 14,78 % | 98,6 %<br>(Thioltitr.) | (Schmelzpunkt:<br>75°C) | 85 % | 93 % | 92% | B | 46 % |
| 1,3-Diaminopropan<br><br>N-(3-Aminopropyl)-2-mercapto-<br>propionsäureamid | C: 44,41 %, H: 8,70 %<br>N: 17,27 %, S 19,76 %<br>C: 44,25 %, H: 8,67 %<br>N: 16,95 %, S: 20,01 % | 98,9 %<br>(Thioltitr.) | (Schmelzpunkt:<br>143°C) | 67 % | 82 % | 88% | B | 79 % |
| Thiomilchsäure als Vergleich | | | | 57 % | 50 % | 70 % | | |

**Beispiel 2: Dauerverformungsmittel für gefärbtes Haar**

| | |
|---|---|
| 13,2 g | N-(2-Hydroxypropyl)-2-mercaptopropionsäureamid |
| 0,4 g | Ammoniak (25%-ige wäßrige Lösung) zur pH-Einstellung |
| 2,0 g | Ammoniumhydrogencarbonat |
| 2,0 g | Isopropanol |
| 1,0 g | Isooctylphenol, oxethyliert mit 10 Mol Ethylenoxid |
| 1,0 g | Poly(dimethyldiallylammoniumchlorid) |
| 0,3 g | Parfümöl |
| 0,1 g | Vinylpyrrolidon/Styrol-Mischpolymerisat (Antara® 430 der GAF Corp.; New York/USA) |
| 80,0 g | Wasser |
| 100,0 g | |

Der pH-Wert dieses Mittels liegt bei 7,0 bis 7,5.

Durch Farbbehandlungen vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 45 Grad Celsius erwärmt. Anschließend wird die Abdeckung entfernt, das Haar mit Wasser gespült und mit 100 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und sodann getrocknet.
Als Ergebnis dieser Behandlung wird eine gleichmäßige, elastische und dauerhafte Verformung der Haare erhalten.

**Beispiel 3: Dauerwellverformungsmittel für normales Haar**

| | |
|---|---|
| 18,0 g | N-(2-Hydroxypropyl)-2-mercaptopropionsäureamid |
| 8,9 g | Ammoniak (25%-ige wäßrige Lösung) |
| 5,0 g | Ammoniumhydrogencarbonat |
| 4,0 g | Harnstoff |
| 2,4 g | Monoethanolamin |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat (Antara® 430 der GAF Corp.; New York/USA) |
| 59,1 g | Wasser |
| 100,0 g | |

Der pH-Wert dieses Mittels beträgt 8,4.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

**Beispiel 4: Dauerwellverformungsmittel für normales Haar**

| | |
|---|---|
| 18,0 g | N-(1-Hydroxymethylpropyl)-2-mercaptopropionsäureamid |

(fortgesetzt)

| | |
|---|---|
| 8,9 g | Ammoniak (25%-ige wäßrige Lösung) zur pH-Einstellung |
| 5,0 g | Ammoniumhydrogencarbonat |
| 2,0 g | D-Glucose |
| 2,4 g | Ammoniak |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat (Antara® 430 der GAF Corp.; New York/USA) |
| 61,1 g | Wasser |
| 100,0 g | |

Der pH dieses Mittels liegt bei 8,0 bis 8,5.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

**Patentansprüche**

1. Mittel zur dauerhaften Verformung von Haaren, **dadurch gekennzeichnet, daß** es als keratinreduzierenden Wirkstoff eine Verbindung der allgemeinen Formel

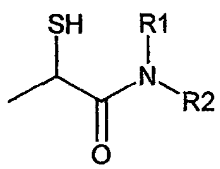

(I)

enthält, wobei $R_1$ und $R_2$ unabhängig von einander jeweils die Bedeutung haben H, geradkettiger oder verzweigter Alkylrest mit 1 - 4 C-Atomen, geradkettiger oder verzweigter Mono- oder Dihydroxyalkylrest mit 2 - 4 C-Atomen, geradkettiger oder verzweigter Alkoxyalkylrest oder Alkylaminoalkylrest mit 2 bis 8 Kohlenstoffatomen, Morpholinoalkyl mit 2 - 3 C-Atomen im Alkylrest, unter der Voraussetzung, daß $R_1$ und $R_2$ nicht gleichzeitig H bedeuten, oder $R_1$ und $R_2$ gemeinsam mit dem Stickstoffatom einen fünf- oder sechsgliedrigen heterocyclischen Ring bilden, der noch ein weiteres Heteroatom enthalten kann und mit ein oder zwei OH- und/oder Methyl-Gruppen substituiert sein kann.

2. Mittel nach Anspruch 1, **dadurch gekennzeichnet, daß** in der Formel (I) $R_1$ und $R_2$ unabhängig voneinander die Bedeutung H , $-CH_3$ , $-CH_2CH_2CH_3$ , $-CH(CH_3)_2$, $-CH_2CH_2OH$, $-CH_2CH(OH)CH_3$ , $-CH(CH_2CH_3)(CH_2OH)$ , $-CH_2CH_2CH_2N(CH_3)_2$ , $-CH_2CH_2OCH_3$ , $-CH_2CH_2CH_2OCH_3$ , $-CH_2CH_2N(CH_2CH_3)_2$ , $-CH_2CH_2N(CH_3)_2$ oder Morpholinoalkyl mit 2 - 3 C-Atomen im Alkylrest haben, unter der Voraussetzung, daß $R_1$ und $R_2$ nicht gleichzeitig H bedeuten.

3. Mittel nach einem der Ansprüche 1 oder 2, **dadurch gekennzeichnet, daß** die Verbindung der Formel (I) in dem gebrauchsfertigen Mittel in einer Menge von 3 bis 28 Gewichtsprozent enthalten ist.

4. Mittel nach einem der Ansprüche 1 bis 3, **dadurch gekennzeichnet, daß** der pH-Wert des gebrauchsfertigen Mittels 6,5 bis 9,5 beträgt.

5. Mittel nach einem der Ansprüche 1 bis 4, **dadurch gekennzeichnet, daß** es vor der Anwendung durch Vermischen von zwei oder drei Komponenten erhalten wird.

6. Verfahren zur dauerhaften Verformung von Haaren, bei dem man das Haar bevor und/oder nachdem man es in der gewünschten Form festhält, mit einem Verformungsmittel behandelt, mit Wasser spült, oxidativ nachbehandelt, erneut mit Wasser spült, gegebenenfalls zur Wasserwelle legt und sodann trocknet, **dadurch gekennzeichnet, daß** man als Verformungsmittel ein Mittel nach einem der Ansprüche 1 bis 5 verwendet.

7. Verfahren nach Anspruch 6, **dadurch gekennzeichnet, daß** man das Verformungsmittel 5 bis 30 Minuten lang einwirken läßt.

8. Verfahren nach Anspruch 6, **dadurch gekennzeichnet, daß** man das Verformungsmittel unter Anwendung von Wärme 5 bis 20 Minuten lang einwirken läßt.

9. Verfahren nach einem der Ansprüche 6 bis 8, **dadurch gekennzeichnet, daß** man das Verformungsmittel in einer Menge von 60 bis 120 Gramm anwendet.

10. Verfahren zur Herstellung von 2-Mercaptopropionsäureamiden der Formel (I), **dadurch gekennzeichnet, daß** man das jeweilige Amin bei einer Temperatur nicht über 30 Grad Celsius mit 2-Mercaptopropionsäure-methylester umsetzt.

## Claims

1. Agent for the permanent shaping of hair, **characterized in that** it comprises, as keratin-reducing active ingredient, a compound of the general formula

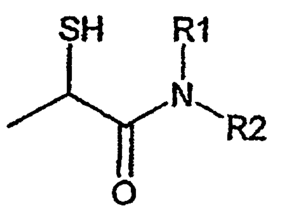

(I)

where $R_1$ and $R_2$, independently of one another, in each case have the meaning H, straight-chain or branched alkyl radical having 1-4 carbon atoms, straight-chain or branched mono- or dihydroxyalkyl radical having 2-4 carbon atoms, straight-chain or branched alkoxyalkyl radical or alkylaminoalkyl radical having 2 to 8 carbon atoms, morpholinoalkyl having 2-3 carbon atoms in the alkyl radical, with the proviso that $R_1$ and $R_2$ are not H at the same time, or $R_1$ and $R_2$, together with the nitrogen atom, form a five- or six-membered heterocyclic ring which can also contain a further hetero atom and can be substituted by one or two OH and/or methyl groups.

2. Agent according to Claim 1, **characterized in that**, in the formula (I), $R_1$ and $R_2$, independently of one another, have the meaning H, $-CH_3$, $-CH_2CH_2CH_3$, $-CH(CH_3)_2$, $-CH_2CH_2OH$, $-CH_2CH(OH)CH_3$, $-CH (CH_2CH_3) (CH_2OH)$, $-CH_2CH_2CH_2N(CH_3)_2$, $-CH_2CH_2OCH_3$, $-CH_2CH_2CH_2OCH_3$, $-CH_2CH_2N(CH_2CH_3)_2$, $-CH_2CH_2N(CH_3)_2$ or morpholinoalkyl having 2-3 carbon atoms in the alkyl radical, with the proviso that $R_1$ and $R_2$ are not H at the same time.

3. Agent according to Claim 1 or 2, **characterized in that** the compound of the formula (I) is present in the ready-to-use agent in an amount of from 3 to 28 per cent by weight.

4. Agent according to one of Claims 1 to 3, **characterized in that** the pH of the ready-to-use agent is 6.5 to 9.5.

5. Agent according to one of Claims 1 to 4, **characterized in that** it is obtained by mixing two or three components prior to use.

6. Method for the permanent shaping of hair in which the hair, before and/or after it is held in the desired shape, is treated with a shaping agent, rinsed with water, oxidatively after-treated, rinsed again with water optionally arranged in a water wave and then dried, **characterized in that** the shaping agent used is an agent according to one of Claims 1 to 5.

7. Method according to Claim 6, **characterized in that** the shaping agent is left to act for 5 to 30 minutes.

8. Method according to Claim 6, **characterized in that** the shaping agent is left to act for 5 to 20 minutes under the application of heat.

9. Method according to one of Claims 6 to 8, **characterized in that** the shaping agent is used in an amount of from 60 to 120 grams.

10. Method of preparing 2-mercaptopropionamides of the formula (I) **characterized in that** the respective amine is reacted with methyl 2-mercaptopropionate at a temperature not exceeding 30 degrees Celsius.

## Revendications

1. Composition pour la mise en forme permanente des cheveux, **caractérisée en ce qu'**elle contient en tant que substance active réduisant la kératine un composé de formule générale

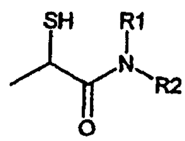

(I)

dans laquelle $R_1$ et $R_2$ représentent, indépendamment l'un de l'autre, H, un radical alkyle à chaîne droite ou ramifiée ayant de 1 à 4 atomes de carbone, un radical mono- ou dihydroxyalkyle à chaîne droite ou ramifiée ayant de 2 à 4 atomes de carbone, un radical alcoxyalkyle ou un radical alkylaminoalkyle, à chaîne droite ou ramifiée, ayant de 2 à 8 atomes de carbone, un radical morpholinoalkyle à 2 ou 3 atomes de carbone dans le fragment alkyle, étant entendu que $R_1$ et $R_2$ ne représentent pas simultanément H, ou $R_1$ et $R_2$ forment ensemble avec l'atome d'azote un cycle hétérocyclique à cinq ou six chaînons, qui peut encore contenir un autre hétéroatome et peut être substitué par un ou deux groupes methyle et/ou OH.

2. Composition selon la revendication 1, **caractérisée en ce que** dans la formule (I) $R_1$ et $R_2$ représentent, indépendamment l'un de l'autre, H, $-CH_3$ $-CH_2CH_2CH_3$, $-CH(CH_3)_2$, $-CH_2CH_2OH$, $-CH_2CH(OH)CH_3$, $-CH\ (CH_2CH_3)(CH_2OH)$, $-CH_2CH_2CH_2N(CH_3)_2$, $-CH_2CH_2OCH_3$, $-CH_2CH_2CH_2OCH_3$, $-CH_2CH_2N(CH_2CH_3)_2$, $-CH_2CH_2N(CH_3)_2$ ou un groupe morpholinoalkyle ayant 2 ou 3 atomes de carbone dans le fragment alkyle, étant entendu que $R_1$ et $R_2$ ne représentent pas simultanément H,

3. Composition selon la revendication 1 ou 2, **caractérisée en ce que** le composé de formule (I) est contenu en une quantité de 3 à 28 % en poids dans la composition prête à l'emploi.

4. Composition selon l'une quelconque des revendications 1 à 3, **caractérisée en ce que** le pH de la composition prête à l'emploi est de 6,5 à 9,5.

5. Composition selon l'une quelconque des revendications 1 à 4, **caractérisée en ce qu'**elle est obtenue avant l'emploi, par mélange de deux ou trois composants.

6. Procédé pour la mise en forme permanente des cheveux, dans lequel les cheveux, avant et/ou après avoir été maintenus sous la forme désirée, sont traités par une composition de mise en forme, rincés à l'eau, soumis à un après-traitement par oxydation, rincés à nouveau à l'eau, éventuellement mis en plis et ensuite séchés, **caractérisé en ce qu'**on utilise comme composition de mise en forme une composition selon l'une quelconque des revendications 1 à 5.

7. Procédé selon la revendication 6, **caractérisé en ce qu'**on laisse agir la composition de mise en forme pendant 5 à 30 minutes.

8. Procédé selon la revendication 6, **caractérisé en ce qu'**on laisse agir la composition de mise en forme, avec application de chaleur, pendant 5 à 20 minutes.

9. Procédé selon l'une quelconque des revendications 6 à 8, **caractérisé en ce que** la composition de mise en forme est utilisée en une quantité de 60 à 120 grammes.

10. Procédé pour la préparation de 2-mercaptopropionamides de formule (I), **caractérisé en ce qu'**on fait réagir l'amine respective avec du 2-mercaptopropionate de méthyle à une température n'excédant pas 30°C.